# EUROPÄISCHE PATENTSCHRIFT

(11) **EP 0 628 544 B1**
(45) Veröffentlichungstag und Bekanntmachung des Hinweises auf die Patenterteilung: **27.12.1996**
(21) Anmeldenummer: 94108407.1
(22) Anmeldetag: 01.06.1994
(51) Int. Cl.: C07C 403/16

(54) **Herstellung von beta-Jonon**
Process for the preparation of beta-ionone
Procédé de préparation de bêta-ionone

(30) Priorität: 11.06.1993 CH 1759/93
(43) Veröffentlichungstag der Anmeldung: 14.12.1994
(73) Patentinhaber: F. HOFFMANN-LA ROCHE AG, 4002 Basel (CH)
(72) Erfinder: Steiner, Kurt, CH-4656 Starrkirch (CH); Ertel, Herwig, D-63762 Grossostheim (DE); Tiltscher, Helmut, D-81739 München (DE)
(74) Vertreter: Kellenberger, Marcus, Dr.

(56) Entgegenhaltungen:
- DE-A- 3 431 131
- CEREAL FOODS WORLD, Bd. 36, Nr. 11, 1991 Seiten 935 - 937, E. S. CARBONELL: "Extraction of Flavors with Supercritical Carbon Dioxide"

## Beschreibung

Die vorliegende Erfindung betrifft ein Verfahren zur Herstellung von β-Jonon aus Pseudojonon in einem zweiphasigen Lösungsmittelsystem.

Eine Vielzahl bedeutender technischer Synthesen erfolgt in heterogenen Flüssig-Flüssig(Zweiphasen)-Lösungsmittelsystemen, wobei die Umsetzung entweder direkt an der Phasengrenze oder in der Bulkphase des Aufnehmers stattfindet.

Technische Beispiele für reaktive, heterogene Flüssig-Flüssig-Lösungsmittelsysteme finden sich unter anderem bei zentralen Zwischenstufen der Synthese von Vitamin A. Hierbei sind Reaktanden und Produkte über zwei flüssige Phasen verteilt.

Technische Synthesen werden heutzutage auch dann systematisch modifiziert, wenn sie in Konkurrenz zu bestehenden und in der Produktion ausgereiften Prozessen stehen. Vor allem der verstärkten Bedeutung eines produkt- und produktionsintegrierten Umweltschutzes kann meist nicht allein durch apparative Neuerungen Rechnung getragen werden. Vielmehr liegt in der Umstellung des stofflichen Systems, sofern dies möglich ist, häufig ein viel grösseres, nutzbares Potential. Die physiologische Bedenklichkeit und die ökologischen Probleme, die mit den im technischen Prozess der Vitamin A-Synthese verwendeten chlorierten, organischen Lösungsmitteln verbunden sind, geben die Motivation zur Entwicklung eines alternativen Verfahrenskonzeptes. Im technischen Prozess wird auch aus wirtschaftlichen und ebenso aus ökologischen Erwägungen eine Reduktion der Einsatzmenge an Schwefelsäure und damit eine Verringerung der im Prozess anfallenden, organisch belastenden Dünnsäure angestrebt, und eine derartige Bestrebung stellt ebenfalls eine Motivation dar.

Ziel der technischen Synthese von Vitamin A ist es, die Vitamin A-Struktur mit ihren 20 Kohlenstoffatomen aus leicht zugänglichen Bausteinen, z.B. aus der Petrochemie, aufzubauen. Mit β-Jonon, einem zyklischen Terpenketen [4-(2,6,6-Trimethyl-1-cyclohexen-1-yl)-3-buten-2-on], steht bereits ein Molekül zur Verfügung, das 13 Kohlenstoffatome, einschliesslich des endständigen C₆-Ringes in der dem Vitamin A entsprechenden Konfiguration, enthält. Alle grosstechnischen Verfahren verlaufen deshalb über β-Jonon als Zwischenstufe.

Die Isler'sche Synthese (Roche) von 1948, deren Schlüsselschritt auf der Verknüpfung eines aus β-Jonon entstehenden C₁₄- mit einem C₆-Baustein beruht, kann bis heute als eines der ökonomisch erfolgreichsten Verfahren der Totalsynthese von Vitamin A betrachtet werden. In der technischen Realisierung umfasst diese Synthese 11 Stufen, von denen die zweite aus der Zyklisierung von Pseudojonon unter Einwirkung von Schwefelsäure zu β-Jonon besteht. β-Jonon wird über die Glycidestersynthese in den C₁₄-Baustein 2-Methyl-4-(2,6,6-trimethyl-1-cyclohexen-1-yl)-2-buten-1-al (den "C₁₄-Aldehyd") übergeführt, der seinerseits mit dem in drei Reaktionsschritten synthetisierten C₆-Baustein 3-Methyl-pent-2-en-4-in-1-ol über eine Grignardreaktion verknüpft wird. Aus dem so entstandenen Oxenin fällt nach partieller Lindlarhydrierung, Acetylierung mit Essigsäureanhydrid, Dehydratisierung und Umlagerung das Vitamin A-Acetat in rohem Zustand an. Nach Reinigung durch Kristallisation und Umesterung mit Methylpalmitat bildet sich Vitamin A-Palmitat, das marktfähige Endprodukt der grosstechnischen Synthese.

Die zentrale Verbindung sämtlicher Vitamin A-Synthesen ist β-Jonon, das sich unter Einfluss starker Protonensäuren durch eine Ringschlussreaktion aus Pseudojonon (6,10-Dimethylundeca-3,5,9-trien-2-on) gewinnen lässt. Bei dieser Ringschlussreaktion entsteht gleichzeitig auch α-Jonon [4-(2,6,6-Trimethyl-2-cyclohexen-1-yl)-3-buten-2-on], das sich lediglich durch die Lage der Doppelbindung im Ring unter Ausbildung eines asymmetrischen Kohlenstoffatoms mit (R)-Konfiguration vom isomeren β-Jonon unterscheidet. α-Jonon wird hauptsächlich in der Riechstoffindustrie als Vorstufe synthetischer Duftstoffe verwendet.

Die Verwendung chlorierter Kohlenwasserstoffe als Lösungsmittel ist bei organischen Synthesen weit verbreitet. Die ausserordentliche Lösekraft von Methylenchlorid nicht nur für terpenoide Verbindungen, sondern auch für einen Grossteil der bei der Synthese entstehenden polymeren Nebenprodukte begründet seinen Einsatz in der technischen Vitamin A-Produktion. Zudem begünstigt die hohe Dichte von Methylenchlorid die Dispergierung der Schwefelsäure bei der Zyklisierung von Pseudojonon, die eine hohe Stoffaustauschrate im zweiphasigen System ermöglicht. Gleichzeitig wird jedoch die nachfolgende Abtrennung der schwefelsauren Phase erschwert. Wie oben bereits angedeutet, steht die Verwendung von chlorierten Lösungsmitteln, wie beispielsweise Methylenchlorid, immer stärker im Blickpunkt der ökologischen Diskussion. Trotz Wiederaufarbeitung des Lösungsmittels lassen sich aufgrund des hohen Dampfdrucks von Methylenchlorid Leckverluste in technischen Anlagen nicht vermeiden. Freiwerdende chlorierte Kohlenwasserstoffe stehen im Verdacht, Veränderungen im Aufbau der Atmosphäre verursachen zu können. Zusätzlich wird eine krebserregende Wirkung dieser Lösungsmittel vermutet, so dass ihre Verwendung bei der Herstellung von pharmazeutischen Produkten und Nahrungsmitteln nicht zuletzt aus psychologischen Gesichtspunkten bedenklich ist. Trotz der Vorteile, die chlorierte Kohlenwasserstoffe im technischen Betrieb bieten, wird aus diesen Gründen intensiv nach einem Ersatz durch alternative Lösungsmittel und Verfahren gesucht.

In der technischen Grossanlage wird Pseudojonon kontinuierlich mit hochkonzentrierter, z.B. 98%iger, Schwefelsäure vollständig umgesetzt, wobei β-Jonon mit einer Ausbeute von etwa 90% anfällt. Das Pseudojonon wird dabei über Dosierpumpen aus Zwischenlagertanks entnommen und in Methylenchlorid gelöst. Das so verdünnte Edukt wird in der Reaktionszone intensiv mit Schwefelsäure im Gewichtsverhältnis etwa 1:2 (Edukt:Säure) vermischt. Die Säure ist nur zu einem geringen Teil in Methylenchlorid löslich, so dass sich ein zweiphasiges Flüssig-Flüssig-System bildet, bei dem die Umsetzung an der Phasengrenze eingeleitet wird. Das Edukt Pseudojonon reagiert an der Oberfläche der dispergierten Schwefelsäuretropfen. Die entstehenden Produkte bleiben in der sauren Phase gebunden. Die freigesetzte Wärme der stark exothermen Umsetzung wird durch Vorkühlung des Pseudojonon/Methylenchlorid-Stromes und intensive Kühlung im Reaktor abgeführt, um eine Temperatur von 0 bis 5°C, insbesondere von etwa 0°C, aufrechtzuerhalten. Da β-Jonon in Kontakt mit Schwefelsäure mit zunehmender Reaktionszeit hochmolekulare, polymere Nebenprodukte bildet, ist es notwendig die Weiterreaktion zu unterbinden. In einer sogenannten Quenchstufe wird durch Zudosieren von Wasser die Schwefelsäure so weit verdünnt, und zwar normalerweise auf eine etwa 18%ige wässrige Lösung, dass unter gleichzeitiger Trennung der entstehenden organischen und der sauren, wässrigen Phase die Umsetzung abgebrochen wird. Die freigesetzte Verdünnungsenthalpie muss dabei abgeführt werden. Da es zudem aus wirtschaftlichen und ökologischen Gründen äusserst wünschenswert ist, die Schwefelsäure zwecks Wiederverwendung wieder in das Reaktionssystem einzuleiten, muss die stark verdünnte wässrige Schwefelsäurelösung gereinigt und aufkonzentriert werden, was infolge des benötigten Grads des Aufkonzentrierens (etwa 18% bis auf etwa 98%) energetisch sehr aufwendig und kostspielig ist.

Aus den obigen Ausführungen ist es klar, dass ein dringendes Bedürfnis besteht, das im Betriebsverfahren zur Herstellung von β-Jonon bisher verwendete Lösungsmittel Methylenchlorid durch ein geeigneteres zu ersetzen, welches die obenerwähnten Nachteile von Methylenchlorid nicht aufweist. Es wurde nun überraschenderweise gefunden, dass die Verwendung verflüssigten (unter Druck stehenden) Kohlendioxids diesem Bedürfnis entspricht. Das erfindungsgemässe Verfahren zur Herstellung von β-Jonon durch die mit Schwefelsäure katalysierte Zyklisierung von Pseudojonon in einem aus konzentrierter Schwefelsäure und einem mit Wasser im wesentlichen nicht mischbaren zweiten Lösungsmittel bestehenden zweiphasigen Lösungsmittelsystem ist dadurch gekennzeichnet, dass das zweite Lösungsmittel unter Druck stehendes flüssiges Kohlendioxid ist.

Bei dem erfindungsgemässen Verfahren handelt es sich im anfänglichen Reaktionsschritt um die Protonierung des Edukts Pseudojonon, das sich hauptsächlich gelöst in der Kohlendioxidphase befindet, durch Schwefelsäure, die als sogenannter Reaktionsvermittler fungiert. Der Protonierung folgt innert kürzester Zeit die eigentliche Umwandlung des Edukts zum erwünschten Produkt β-Jonon, und zwar durch Zyklisierung des protonierten Pseudojonons. Diesbezügliche Experimente haben gezeigt, dass die Protonierung und die darauf folgende Zyklisierung an den Phasengrenzen zwischen dem flüssigen Kohlendioxid und der Schwefelsäurephase geschehen. Danach bleibt das (noch) nicht umgesetzte Pseudojonon in der Kohlendioxidphase, während das Produkt, das sich im protonierten Zustand befindet, in die Schwefelsäurephase aufgenommen wird. Erst durch die Zugabe von Eiswasser, gekühlter wässriger Lauge, insbesondere wässriger Ammoniumhydroxidlösung einer Konzentration von 2 bis 4 Mol/L (7 bis 14%ig) bei 0 bis 10°C, oder gekühlter, stark verdünnter Schwefelsäure, insbesondere 30 bis 40%iger Schwefelsäure bei 0 bis 10°C, zur Reaktionsmischung kann die Deprotonierung dieses Produktes erfolgen. Dabei wird die Umsetzung abgestoppt.

Nun lassen die experimentellen Ergebnisse der Umsetzung von Pseudojonon mit konzentrierter Schwefelsäure ein komplexeres Reaktionsgeschehen erkennen. Mit Schwefelsaure reagiert Pseudojonon nämlich auf parallelen Wegen zu α- und β-Jonon, wobei das Mengenverhältnis dieser beiden Produkte von den jeweils eingestellten Reaktionsbedingungen abhängt, wie beispielsweise Konzentration der Schwefelsäure, Reaktionstemperatur(bereich), Mengenverhältnis von Edukt und Schwefelsäure, und Reaktionsdauer. Ferner ist unter der Wirkung von Schwefelsäure die direkte Umlagerung von α-Jonon zu β-Jonon möglich. Darüber hinaus tendiert sowohl das Edukt Pseudojonon wie auch das erwünschte Produkt β-Jonon, und auch noch das als Nebenprodukt des erfindungsgemässen Verfahrens betrachtete α-Jonon, zu unerwünschter Polymerenbildung, und zwar insbesondere unter der Einwirkung von hochkonzentrierter Schwefelsäure: sowohl der Kontakt protonierter Verbindungen mit unprotoniertem Edukt (Pseudojonon) aus der Kohlendioxidphase als auch der Angriff überschussiger Schwefelsäure auf bereits gebildete Produkte ist möglich. Die Polymerverbindungen unterschiedlicher chemischer Zusammensetzung treten bereits nach den kürzesten Reaktionszeiten, wie beispielsweise 2,5 Minuten, in deutlicher Menge auf, und ihr Anteil im Gesamtprodukt nimmt während der Umsetzung stetig zu und ist unter anderem auch abhängig von der Säurekonzentration und von der Temperatur des Mischens der beiden Phasen. Diese Beobachtungen führen zu dem Schluss, dass sowohl die Einflussparameter des chemischen Reaktionssystems als auch die Reaktionsführung auf eine optimale Ausbeute des erwünschten Produktes β-Jonon abzustimmen sind. Während durch die an der Reaktion teilnehmende Schwefelsäure das Mengenverhältnis der parallel entstehenden Produkte α- und β-Jonon bestimmt wird, ist es Aufgabe der Reaktionsführung, die Umsetzung vor Ueberschreitung des Anteilmaximums an β-Jonon abzubrechen. Die Verweilzeit der umgesetzten Komponenten im Reaktionsraum erhält damit entscheidende Bedeutung, und kann so gewählt werden, dass u.a. die Entstehung polymerer Nebenprodukte zurückgedrängt wird.

Da im erfindungsgemässen Verfahren das Kohlendioxid im verflüssigten Zustand vorliegen soll, steht es zwangsläufig unter relativ hohem Druck. Im allgemeinen liegt der Druck, unter welchem das Verfahren durchgeführt wird, im Bereich von 50 bar bis 150 bar, vorzugsweise im Bereich von 80 bar bis 120 bar.

Was die Konzentration der Schwefelsäure betrifft, wird beobachtet, dass im allgemeinen eine relativ hohe Konzentration zu der gewünschten ausgeprägten Bildung von β-Jonon führt, während die Verwendung relativ verdünnter (weniger als 60-prozentiger) Schwefelsäure (praktisch) keine Zyklisierung des Pseudojonons initiert. Bei der Verwendung von 75- bis 85-prozentiger Schwefelsäure entsteht vornehmlich unerwünschtes α-Jonon. Im allgemeinen beträgt die Konzentration der im erfindungsgemässen Verfahren verwendeten Schwefelsäure mindestens 85-Prozent. Vorzugsweise verwendet man Schwefelsäure der Konzentration zwischen 95- und 98-Prozent.

Das molare bzw. Massen(Gewichts)-Verhältnis von Schwefelsäure zu Pseudojonon stellt einen weiteren Reaktionsparameter dar. Es ist festgestellt worden, dass Pseudojonon im zweiphasigen System verflüssigtes Kohlendioxid (als Eduktlösungsmittel)/Schwefelsäure erst bei einem molaren Verhältnis Schwefelsäure:Pseudojonon grösser als 2:1 (Massenverhältnis 1:1) bei einer Reaktionstemperatur von 5°C vollständig abreagiert. Anders ausgedrückt, ist mindestens die doppelte molare Menge an Schwefelsäure bei dieser Temperatur notwendig, um eine vollständige Protonierung der basischen Zentren des Pseudojonons zu erreichen. Zuviel Säure (und/oder zu konzentrierte Säure) führt zu übermässiger Bildung von polymeren Nebenprodukten, was auch bei zuwenig Schwefelsäure der Fall ist. Eine maximale Ausbeute an β-Jonon wird beispielsweise bei ca. 5°C erreicht, wenn das molare Verhältnis Schwefelsäure:Pseudojonon 3:1 (Massenverhältnis 1,5:1) beträgt. Selbstverständlich übt hier die Reaktionstemperatur einen Einfluss auf die Ausbeute bei einem bestimmten Molverhältnis aus, und zwar im Sinne, dass höhere Reaktionstemperaturen, z.B. Temperaturen oberhalb von 15°C, zu kleineren Ausbeuten infolge erhöhter Polymerenbildung führen. Geeigneterweise wird das erfindungsgemässe Verfahren bei einem molaren Verhältnis Schwefelsäure:Pseudojonon im Bereich von 2:1 bis 5:1, vorzugsweise von 2,5:1 bis 3,5:1, durchgeführt.

Das Massenverhältnis von Kohlendioxid zu Pseudojonon beträgt zweckmässigerweise von 100:1 bis 100:5.

Bei der Bestimmung der Reaktionstemperatur muss unter anderem berücksichtigt werden, dass zu hohe Temperaturen zu einer übermässigen Polymerenbildung, während zu niedrige zu einem unzufriedenstellend langsamen Reaktionsablauf führen. Im allgemeinen wird das erfindungsgemässe Verfahren bei Temperaturen von -15°C bis +15°C durchgeführt. Vorzugsweise verwendet man den Temperaturbereich von -5°C bis +10°C, insbesondere denjenigen von 0°C bis 5°C, wobei der engere Bereich besonders gute Ausbeuten an β-Jonon bei zufriedenstellender Reaktionsgeschwindigkeit und keiner übermässigen Bildung von unerwünschten polymeren Nebenprodukten ermöglicht.

Unter Berücksichtigung der sonstigen Reaktionsbedingungen und des Wunsches auf eine höchstmögliche Ausbeute an β-Jonon bei minimaler Erzeugung von unerwünschten Nebenprodukten, wie α-Jonon und polymeren Verbindungen, dauert die erfindungsgemäss verwirklichte Ueberführung von Pseudojonon in β-Jonon in der Reaktionsstufe (vor der Quenchstufe) etwa 10 Minuten bis etwa 40 Minuten.

Während der erfindungsgemäss durchgeführten Reaktion ist ein intensives Durchmischen der Schwefelsäure- und der Kohlendioxidphase vorteilhaft, da die Protonierung und nachfolgende Zyklisierung von Pseudojonon unter der Einwirkung von Schwefelsäure an der Phasengrenze stattfindet. Auf diese Weise, d.h. durch Erzeugung einer grösstmöglichen Stoffaustauschfläche bei kleinen Tropfenradien, kann die bestmögliche Nutzung der eingesetzten Säuremenge erzielt und die Reaktion effizient durchgeführt werden.
Das obenerwähnte, nach der Reaktionsstufe eingeschaltete Quenchen, welches ein rasches Verdünnen der Reaktionsmischung, daraus resultierende Deprotonierung des Produktes, Trennung der Reaktionsmischung in eine wässrige saure und eine organische Phase (die Produktphase) sowie die Zurückdrängung der unerwünschten Polymerisation ermöglicht, erfolgt zweckmässigerweise durch Zugabe von Eiswasser, gekühlter, wässriger Lauge oder gekühlter, stark verdünnter Schwefelsäure. Das Quenchen ermöglicht ein rasches und vollständiges Abstoppen der chemischen Umsetzung unter rascher Abfuhr der entstandenen Wärmemenge. Zum Quenchen wird zweckmässigerweise soviel Flüssigkeit, vorzugsweise Eiswasser, verwendet, dass eine Verdünnung der Schwefelsäurephase auf 40- bis 50-Prozent erreicht wird. Die abgetrennte Produktphase wird zur Isolierung des reinen β-Jonons aufgearbeitet, und zwar zweckmässigerweise dadurch, dass man die Produktphase einer Hochdruckextraktionskolonne zuführt und mit Kohlendioxid als Lösungsmittel extrahiert. Nach Entfernung des Lösungsmittels, welches zum Recycling gelangen kann, wird das erwünschte β-Jonon erhalten. Die verdünnte Schwefelsäurephase wird auf konventionelle Weise, z.B. Filtration oder kontinuierliche Zentrifugation, von organischen Rückständen befreit und anschliessend, ebenfalls auf an sich bekannte Weise, aufkonzentriert, so dass die resultierende hochkonzentrierte Schwefelsäure zum Recycling gelangen kann.

Das erfindungsgemässe Verfahren kann sowohl diskontinuierlich als auch kontinuierlich durchgeführt werden. Eine kontinuierliche Arbeitsweise, wobei u.a. möglichst viel des verflüssigten Kohlendioxids, allfälliges unreagiertes Pseudojonon sowie die aufkonzentrierte Schwefelsäure in das Reaktionssystem zurückgeleitet wird, ist allerdings bevorzugt.

In einer typischen kontinuierlich betriebenen, technischen Anlage, die die Verwendung von unter Druck stehendem flüssigem Kohlendioxid als Eduktlösungsmittel ermöglicht, wird in der Reaktionszone verflüssigtes Kohlendioxid durch ein Dispergierorgan umgewälzt. Pseudojonon wird dem Kreislaufstrom zudosiert und in einem statischen Mischer homogen in der kontinuierlichen Phase gelöst. Ihrerseits wird die Schwefelsäure durch ein Dispergierorgan in der Eduktphase verteilt, und zwar zweckmässigerweise nach Injizieren direkt vor dem Dispergierorgan. Es wird nahezu sofort eine grosse Stoffaustauschfläche durch intensive Durchmischung des Zweiphasensystems erreicht. Auf diese Weise wird ein Vermischungszustand erzeugt, der zwischen den Randfällen der vollständig durchmischten Bulkphasen und der starren, undurchmischten Säuretropfen liegt. Bestmögliche Nutzung der eingesetzten Säuremenge kann durch Erzeugung einer grossen Stoffaustauschfläche bei kleinen Tropfenradien erzielt werden. Allerdings wird durch die an den Tropfenoberflächen gebundenen, protonierten organischen Verbindungen bei Eduktüberschuss Polymerisation nach ionischem Mechanismus begünstigt. Neben der Optimierung der Pseudojononkonzentration in der kontinuierlichen Kohlendioxidphase kann durch eine rasche Abtrennung der sauren Produktphase, beispielsweise indem man die schwerere Produktphase in einem Hydrozyklon einleitet, diese Polymerisation zurückgedrängt werden. Damit wird eine rasche Trennung der organischen, protonierten Verbindungen von unverbrauchtem Pseudojonon ermöglicht und die Bildung hochmolekularer Nebenprodukte zurückgedrängt. Da die Ausbeute an β-Jonon ein zeitliches Maximum durchläuft, ist in der Reaktionszone für eine enge Verweilzeitverteilung zu sorgen. Die bei der exothermen Umsetzung entstehende Wärmemenge muss abgeführt und die Umsetzung vollständig abgebrochen werden. Durch Kühlen des Kreislaufstroms im statischen Mischer ist es möglich, die Wärmeentwicklung der Umsetzung zu beherrschen. Während z.B. in einen Hydrozyklon unverbrauchtes Pseudojonon im Lösungsmittel Kohlendioxid zurückgeführt wird, wird die saure Produktphase abgezogen. Diese kann zur Vervollständigung der Zyklisierüngsreaktion einen Verweilzeitreaktor durchlaufen. Durch die Stoffstromführung in der Reaktionszone (Rückführung des nicht umgesetzten, gelösten Edukts und lineare Führung der Säure/Produkt-Phase) ist es möglich, die angestrebte enge Verweilzeitverteilung der gebildeten Produkte im Reaktionsbereich der Anlage zu realisieren.

Zum vollständigen Abstoppen der chemischen Umsetzung unter rascher Abfuhr der Wärmemenge ist der Reaktionszone eine Quenchzone nachgeschaltet. Dabei muss die komplexe Reaktionsmischung, bestehend aus protonierten, organischen Substanzen und konzentrierter Schwefelsäure, zerstört werden, was durch rasches Verdünnen der Säure erreicht wird. Die Quenchzone kann wiederum als Kreislauf gestaltet werden, wodurch eine intensive Vermischung und Kühlung zur Abfuhr der Verdünnungswärme ermöglicht wird. Die Verdünnung der sauren Produktphase, vorzugsweise auf einen 45%igen Schwefelsäureanteil, führt zur Auftrennung in eine organische Produktphase und in wässrige Schwefelsäure. Die organischen Produkte gelangen zur Aufreinigung. Zumindest ein Teil des Schwefelsäurestroms kann von organischen Rückstanden gereinigt und anschliessend aufkonzentriert werden.

Das Quenchen kann auch durch Zusetzen einer gekühlten, wässrigen Lauge, insbesondere wässrigen Ammoniaks erfolgen, wobei unter Bildung anorganischer Salzlösungen die komplexe Reaktionsmischung ebenfalls aufgetrennt wird. Beim Zudosieren wässrigen Ammoniaks, zweckmässigerweise in einer molaren Konzentration von 2 bis 4 (pro L; 7 bis 14%ig) bei 0 bis 10°C, wird erreicht, dass sich die organischen Verbindungen als leichte Phase abscheiden und das entstehende Ammoniumsulfat in der wässrigen Phase in Lösung bleibt. Salzverkrustungen werden dadurch vermieden. Nach Versetzen der wässrigen Phase mit einem organischen Lösungsmittel, z.B. Aceton, und Eindampfen bleibt Ammoniumsulfat als weisses Pulver zurück. Nach der Filtration werden Wasser und organische Verunreinigungen mit dem Lösungsmittel abgeführt. Die vorher abgezogenen organischen Reaktionsprodukte werden mit flüssigem Kohlendioxid gestrippt, wodurch sich die Jonone von den hochmolekularen Polymerprodukten abtrennen lassen.

Als weitere Variante kann mit gekühlter, stark verdünnter Schwefelsäure, zweckmässigerweise bei 0 bis 10°C und einer Konzentration von 30 bis 40 Gewichtsprozent, gequencht werden.

Nach Durchführung des erfindungsgemässen Verfahrens und der nachfolgenden Isolierung des erwünschten β-Jonons lässt sich durch Entspannen das Kohlendioxid, welches nicht in das System zurückgeleitet wird, rückstandslos aus den Synthesenprodukten entfernen. Als physiologisch inerte Substanz bringt dieses Lösungsmittel darüber hinaus auch im Produktionsprozess selbst keine besonderen Auflagen, so dass aufwendige Schutzmassnahmen im Rahmen einer "good manufacturing performance" für das betroffene Personal entfallen können. Darüber hinaus bietet der Einsatz von Kohlendioxid als Lösungsmittel eine Möglichkeit zur umweltschonenden und damit ökologisch sinnvollen Verwendung dieser in Verbrennungsprozessen überschüssig erzeugten Substanz, zumal sie als Treibhausgas immer stärker in den Mittelpunkt des umweltpolitischen Interesses rückt. Ein weiterer Vorteil des erfindungsgemässen Verfahrens gegenüber der bisherigen Verwendung von chlorierten Kohlenwasserstoffen als Lösungsmittel besteht in der Reduzierung der Menge des Reaktionsvermittlers Schwefelsäure, deren Wiederaufarbeitung oder Entsorgung einen beträchtlichen Teil der Produktionskosten ausmacht. Kohlendioxid besitzt verglichen mit Methylenchlorid eine geringe Löslichkeit für Schwefelsäure. Daher wird ein geringerer Austrag der Säure aus dem Reaktionssystem ermöglicht.

Das erfindungsgemässe Verfahren wird durch das nachfolgende Beispiel veranschaulicht.

### Beispiel

Die für eine kontinuierliche Versuchsdurchführung des erfindungsgemässen Verfahrens verwendete Apparatur wird anhand der nachfolgenden Abbildung dargestellt:

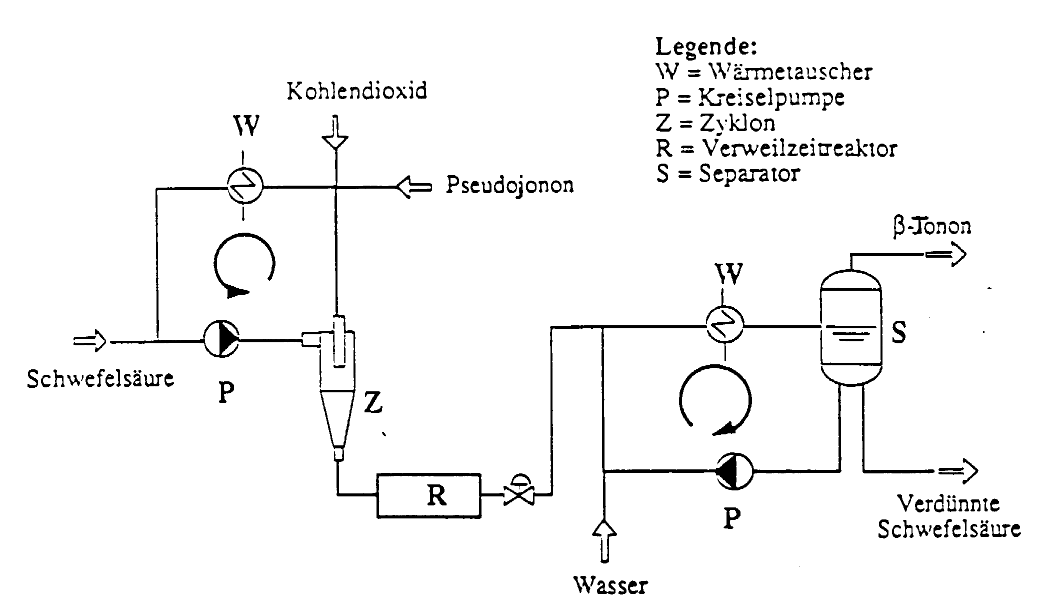

Als Lösungsmittel für das Pseudojonon wird verdichtetes Kohlendioxid (CO₂) eingesetzt. Dem CO₂-Kreisstrom wird kontinuierlich das Edukt Pseudojonon zudosiert und gelöst. Zudem muss die geringe, in der schweren Phase gelöste CO₂-Menge, die somit den Gaskreislauf verlässt, ersetzt werden.

Der so mit Pseudojonon beladene Gasstrom wird vorgekuhlt. Unmittelbar vor der Kreiselpumpe wird konzentrierte Schwefelsäure zudosiert. Die Kreiselpumpe dient einerseits als Förderorgan und andererseits als Dispergierorgan. Die so entstandene Emulsion wird einem Separator (Fluidzyklon) zugeführt und in die leichte Gasphase und in die schwere schwefelsäurehaltige Phase aufgetrennt. Die leichte Phase wird rezirkuliert, die schwere Phase einem Verweilzeitreaktor mit variablem Volumen zugeführt. führt. Nach Ausgang aus dem Verweilzeitreaktor wird die saure Produktphase auf Umgebungsdruck entspannt und durch Zuführen verdünnter Schwefelsäure gequencht. Nach Abführen der Verdünnungswärme wird in einem Separator die verdünnte Schwefelsäure vom Produkt β-Jonon getrennt.

Bei den durchgeführten Versuchen liegt die Durchlaufmenge im Reaktionsstrom im Bereich von 40 bis 60 kg/h. Im Kreisstrom werden zwischen 1 und 5 Gewichtsprozent (Gew.%), in der Regel jedoch 2 Gew.%, Pseudojonon gelöst. Diese Beladung ist nicht durch die Löslichkeit des Pseudojonons in Kohlendioxid, sondern durch die Erwärmung (auf Grund der abzuführenden Protonierungsenergie) des auf 60 kg/h limitierten Reaktionsstroms begrenzt. So hat man bei einer Beladung von 2 Gew.% und einer Durchlaufmenge im Reaktionsstrom von 50 kg/h bereits eine Temperaturerhöhung von etwa 9°C, d.h., dass vor dem Dispergierorgan um diese Temperatur unter die gewünschte Reaktionsstemperatur gekühlt werden muss.

Um die α-Jononbildung effektiv zu limitieren, hat sich gezeigt, dass die Konzentration der Schwefelsäure mindestens 90-Prozent sein soll. Die kontinuierlichen Versuche werden mit 95- bis 98-prozentiger Schwefelsäure durchgeführt, wobei die Ausbeuten bei der 98-prozentigen Schwefelsäure geringfügig bessere Werte geben.

Beim Quenchen mit verdünnter Schwefelsäure auf eine Säurekonzentration von 55-Prozent wird eine Zersetzung des β-Jonons (nach 15 Minuten, bei 20°C) von etwa 4 Gew.% festgestellt. Bei Säurekonzentrationen unter 45-Prozent gibt es eine kaum merkliche Zersetzung. So wird bei den kontinuierlichen Versuchen die Reaktion im allgemeinen durch Herabsetzung der Schwefelsäurekonzentration auf 40% durch Zuleiten von 35-prozentiger Schwefelsäure gequencht. Die Durchlaufmenge des rezirkulierten Schwefelsäurestroms beträgt etwa 16 kg/h.

Die Ergebnisse der Versuche sind in der nachfolgenden Tabelle angegeben:

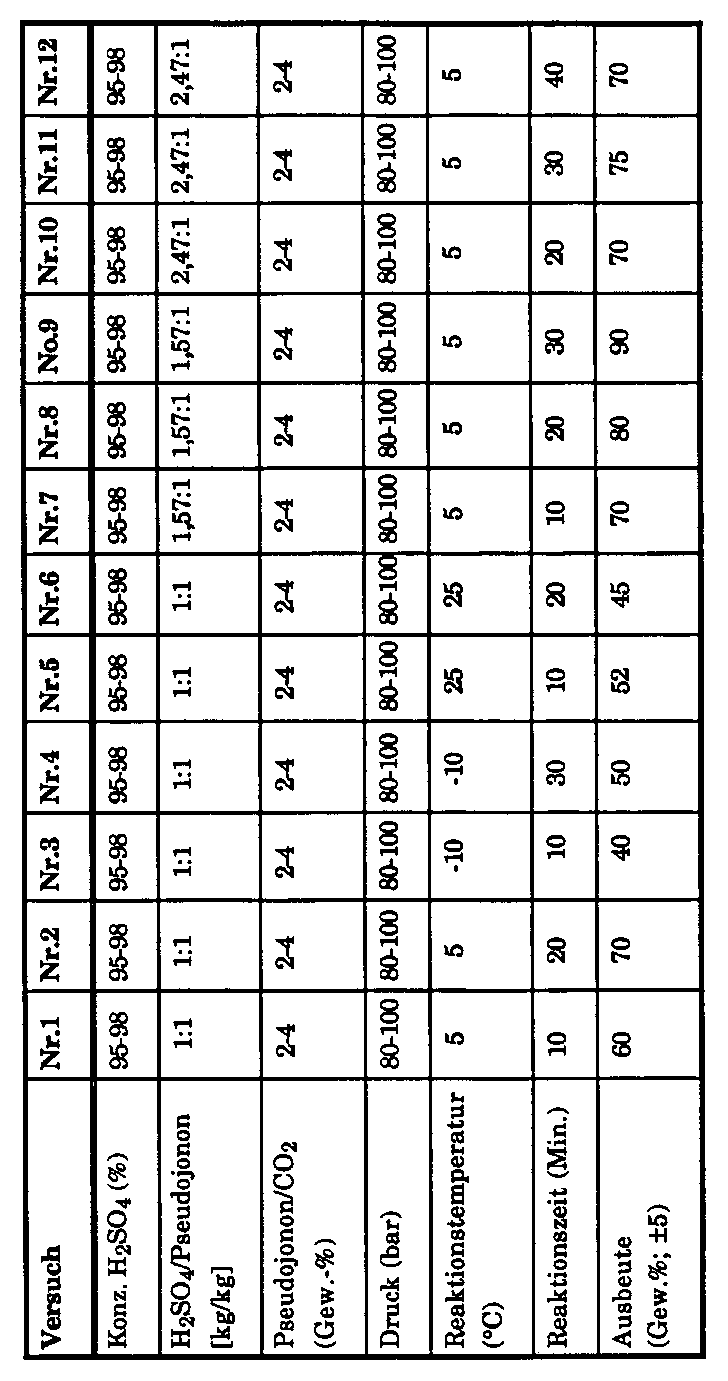

## Patentansprüche

1. Verfahren zur Herstellung von β-Jonon durch die mit Schwefelsäure katalysierte Zyklisierung von Pseudojonon in einem aus konzentrierter Schwefelsäure und einem mit Wasser im wesentlichen nicht mischbaren zweiten Lösungsmittel bestehenden zweiphasigen Lösungsmittelsystem, dadurch gekennzeichnet, dass das zweite Lösungsmittel unter Druck stehendes flüssiges Kohlendioxid ist.

2. Verfahren nach Anspruch 1, dadurch gekennzeichnet, dass es unter einem Druck im Bereich von 80 bar bis 120 bar durchgeführt wird.

3. Verfahren nach Anspruch 1 oder 2, dadurch gekennzeichnet, dass die Konzentration der Schwefelsäure mindestens 85-Prozent, vorzugsweise zwischen 95- und 98-Prozent, ist.

4. Verfahren nach einem der Ansprüche 1 bis 3, dadurch gekennzeichnet, dass es bei einem molaren Verhältnis Schwefelsäure:Pseudojonon im Bereich von 2:1 bis 5:1, vorzugsweise von 2,5:1 bis 3,5:1, durchgeführt wird.

5. Verfahren nach einem der Ansprüche 1 bis 4, dadurch gekennzeichnet, dass es bei Temperaturen von -5°C bis +10°C, vorzugsweise von 0°C bis 5°C, durchgeführt wird.

6. Verfahren nach einem der Ansprüche 1 bis 5, dadurch gekennzeichnet, dass die Reaktion durch Mischen des Reaktionsproduktes mit Eiswasser, gekühlter, wässriger Lauge oder gekühlter, stark verdünnter Schwefelsäure abgebrochen wird.

7. Verfahren nach einem der Ansprüche 1 bis 6, dadurch gekennzeichnet, dass es kontinuierlich durchgeführt wird.

## Claims

1. A process for the manufacture of β-ionone by the sulphuric acid-catalyzed cyclization of pseudoionone in a two-phase solvent system comprising concentrated sulphuric acid and a second solvent essentially immiscible with water, characterized in that carbon dioxide liquefied under pressure is used as the second solvent.

2. A process according to claim 1, characterized in that it is carried out under a pressure in the range of 80 bar to 120 bar.

3. A process according to claim 1 or 2, characterized in that the concentration of the sulphuric acid is at least 85 percent, preferably between 95 and 98 percent.

4. A process according to any one of claims 1 to 3,
characterized in that it is carried out using a molar ratio of sulphuric acid:psuedoionone in the range of 2:1 to 5:1, preferably of 2.5:1 to 3.5:1.

5. A process according to any one of claims 1 to 4,
characterized in that it is carried out at temperatures from -5°C to +10°C, preferably from 0°C to 5°C.

6. A process according to any one of claims 1 to 5,
characterized in that the reaction is stopped by mixing the reaction product with ice-water, cooled aqueous alkali or cooled, strongly diluted sulphuric acid.

7. A process according to any one of claims 1 to 6,
characterized in that it is carried out continuously.

## Revendications

1. Procédé pour la préparation de β-ionone par la cyclisation catalysée par de l'acide sulfurique de pseudoionone dans un système solvant à deux phases consistant en acide sulfurique concentré et en un second solvant pratiquement non miscible à l'eau, caractérisé en ce que le second solvant est du dioxyde de carbone maintenu fluide sous pression.

2. Procédé selon la revendication 1, caractérisé en ce qu'il est mis en oeuvre sous une pression se situant dans l'intervalle de 80 bar à 120 bar.

3. Procédé selon l'une des revendications 1 ou 2, caractérisé en ce que la concentration de l'acide sulfurique est d'au moins 85 pour cent, et de préférence comprise entre 95 et 98 pour cent.

4. Procédé selon l'une quelconque des revendications 1 à 3, caractérisé en ce qu'il est mis en oeuvre avec un rapport molaire acide sulfurique:pseudoionone dans l'intervalle de 2:1 à 5:1, de préférence de 2,5:1 à 3,5:1.

5. Procédé selon l'une quelconque des revendications 1 à 4, caractérisé en ce qu'il est mis en oeuvre à des températures de -5°C à +10°C, de préférence de 0°C à 5°C.

6. Procédé selon l'une quelconque des revendications 1 à 5, caractérisé en ce que la réaction est interrompue par mélange du produit de réaction avec de l'eau glacée, une lessive alcaline aqueuse refroidie ou de l'acide sulfurique fortement dilué, refroidi.

7. Procédé selon l'une quelconque des revendications 1 à 6, caractérisé en ce qu'il est mis en oeuvre en continu.
